# EUROPEAN PATENT APPLICATION

(11) **EP 0 747 094 A2**
(43) Date of publication of application: **11.12.1996**
(21) Application number: 96304337.7
(22) Date of filing: 10.06.1996
(51) Int. Cl.: A61N 5/10

(54) **A process for converting the beam diameter of radioactive rays and radiating unit**

(30) Priority: 08.06.1995 CN 95106841
(71) Applicant: SONG, Shipeng, Guangdong 518042 (CN)
(72) Inventor: SONG, Shipeng, Guangdong 518042 (CN)
(74) Representative: Barlow, Roy James

(57) **Abstract**

The present invention discloses a process for converting the beam diameter of radioactive rays and a radiating unit used in a medical stereotactic radiotherapeutic apparatus. Over a collimator carrier (11) symmetrical about a central axis are distributed a number of sets of collimators (1) of different aperture diameter. The rule of distribution of each set of collimators is the same as that of the radioactive sources (2) in the source carrier (4). The collimator carrier (11) can be rotated according to the requirement of the therapy to bring a set of collimators (1) of a certain aperture diameter in alignment with the radioactive sources (2). Thus it is possible to alter the size of the beam diameter of radioactive rays. The advantages of the invention are convenience in operation, enhancement of the accuracy of positioning and the easiness of putting into practice the automatic control by a computer.

## Description

The present invention relates to a process for converting the beam diameter of radioactive rays, especially a process for converting the beam diameter of radioactive rays in a medical stereotactic radiotherapeutic apparatus. The present invention also relates to a medical radioactive source radiating unit, in particular, the radiating unit in a medical stereotactic radiotherapeutic apparatus.

In the medical stereotactic radiotherapeutic apparatus it is necessary to add a collimator on the outlet end of the radioactive source to form the ray beam of specified diameter. To convert the size of the beam diameter of radioactive rays, the conventional process is to mount a set of unique aperture collimators on a special helmet, the aperture of the collimator on the helmet being made in alignment with the passageway of the radioactive source. In use, one selects a set of collimators having an aperture diameter corresponding to the size of the lesion. For example, the Leksell Gamma knife produced by the Elekta Corporation, Sweden, is provided with four helmets of different types. On each of these helmets is mounted a set of final collimators forming respectively at the site of focus, beams of Gamma rays of 50% dosage and of diameter of 4mm, 8mm, 14mm and 18mm. Furthermore, there is disclosed in Chinese Patent CN-1087551A a rotational coniformly-focused Gamma ray radiating unit. In this first generation rotary Gamma knife developed from this patented technology, there are provided five sets of collimators of different aperture diameters and a helmet. The diameter of the Gamma ray beams is to be adjusted by repositioning of the collimators. In the case of a complicated shape of the lesion, it is necessary to replace manually the helmet or the collimators several times when either of the above-mentioned two processes is being used, so as to guarantee the curative effect. In this way, not only is the operation overelaborate but also positioning and safety protection would be a problem.

The object of the present invention is to improve the conventional process for converting the diameter of the radioactive ray beam so that, during the process of medical treatment, the size of the diameter of the ray beam can be conveniently adjusted. To this end, the present invention will also provide a radiating unit to effect this conversion process.

To achieve the above-mentioned object, the solution of the present invention is as follows: A set of radioactive sources is mounted in the source carrier in such a manner that they are radially in alignment with a common focus on the central axis of the source carrier. Over a collimator carrier symmetrical to a central axis are distributed a number of sets of collimators of different aperture diameter. The rule of distribution for each set of collimators is just the same as that for the radioactive sources. The collimator carrier can rotate relative to the source carrier about its central axis in order to be positioned, and comes into contact with the inner cavity of the source carrier. When a set of collimators of a certain aperture diameter is being selected in accordance with the treatment planning system, the collimator carrier is made to rotate so that the selected collimators comes into register with the radioactive source. When a set of collimators of other aperture diameter is selected, the collimator carrier can be made to rotate again until another selected set of collimators come into contact with the radioactive source, so that it is possible for the diameter of the ray beam to be converted during the medical treatment.

To carry out the above-mentioned process for converting the beam diameter of radioactive rays, a radiating unit can be used which comprises a source carrier, a shielding case, a collimator carrier, a collimator carrier driving unit, and a collimator carrier driving member. In the source carrier, a set of radioactive sources are radially in alignment with a common focus on the central axis of the source carrier. A mandrel is provided in the middle of the collimator carrier. One end of the mandrel is connected with the collimator carrier driving unit via a gear and the other end is fixed in the collimator.

In the radiating unit mentioned above, the connections of the collimator carrier with the source carrier and of the source carrier with the shielding case are effected by bearings. The source carrier is provided with a sleeve which can be rotated relative to the shielding case and the mandrel. One end of the sleeve is fixed on the source carrier and the other end is connected to the source carrier driving unit via the gear. Normally the source carrier rotates at a low speed to perform the function of rotational focusing of the beams at the focus 14. The source carrier and the collimator carrier can move relatively to replace the set of collimators and to act as a shield during non-therapy hours. During the therapeutic process, the source carrier and the collimator carrier are relatively stationary in that they rotate together. The rotation of the collimator carrier and the source carrier is elaborately controlled by the double servo-control system.

A plurality of sets of collimators may be arranged on the collimator carrier according to the rule of distribution of the radioactive sources. No helmet is needed, nor is it necessary to replace the collimators manually. In the process of therapy, the beam diameter of the rays can be converted whenever necessary to change the size of the focus 14. The advantages of the invention are :- simple construction, convenient operation, enhancement of the accuracy of positioning, and the ease of putting into practice the automatic control by computer. Besides, the addition of a set of shielding rods in the collimator carrier would enhance safety protection.

The following is a more detailed description of the present invention in connection with the accompanied drawings and embodiments, in which:
FIGURE 1 is a diagrammatic view of a specific embodiment of the process, according to the present invention, for converting the beam diameter of radioactive rays; and
FIGURE 2 is a structural diagrammatic view of a specific embodiment of a radiating unit for carrying out the process shown in Figure 1.

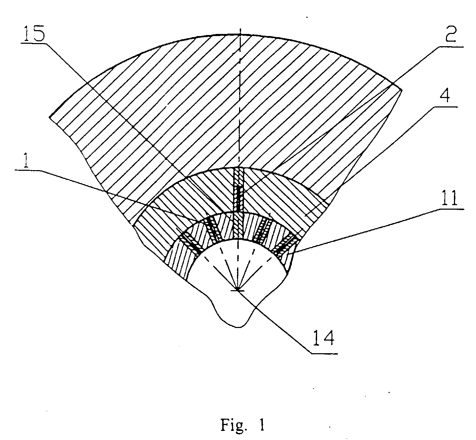

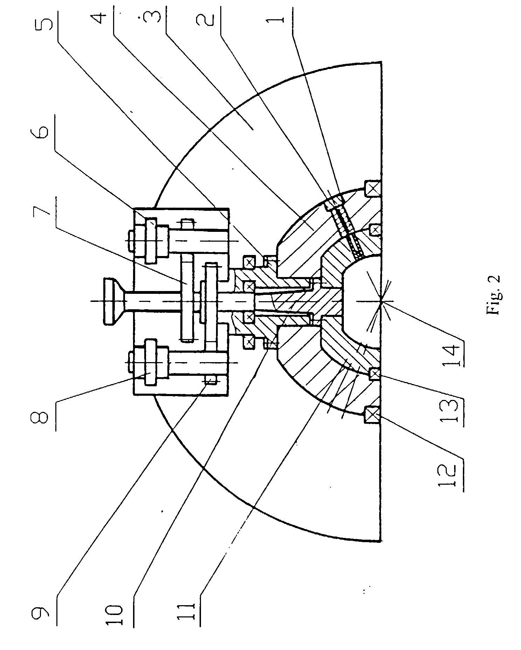

A set of radioactive sources 2 is mounted in a semi-spherical source carrier 4 radially in alignment with the centre 14 of the sphere. Figures 1 and 2 show one source 2 and Figure 1 shows one shielding rod 15 and four collimators 1 of different diameter, associated with that source 2. In Figure 2 the centre lines of other sources (not shown) can be seen.

Over a collimator carrier 11 in the inner cavity of source carrier 4 are distributed several sets of such collimators 1 each set being of different aperture diameter. The rule of distribution for each set of the collimators is the same as that for the radioactive sources. Figure 1 is a partial development view of a theoretical cone. The centre line of a given radioactive source 2 of a set of radioactive sources on the source carrier 4 is located on this theoretical conical surface. The centre lines of four collimators 1 of different inner diameter and the centre line of a shielding rod 15, all shown in Figure 1, are arranged on the same conical surface of the collimator carrier 11 at a certain angular spacing. When, according to the treatment planning system a set of collimators of a certain aperture diameter is required the collimator carrier 11 is rotated to bring the selected different set of collimators into register with the radioactive sources 2. The radioactive rays are being emitted along the collimators. When a set of collimators of other aperture diameter is selected, the collimator carrier 11 can be made to rotate again about the central axis until the selected other set of collimators comes into register with the set of radioactive sources 2. In this way, the conversion of the beam diameter of the rays can be effected. In this embodiment there are 30 of the radioactive sources 2 and the number of the collimators on the collimator carrier 11 is 4 x 30=120. Moreover, during non-therapy hours, when the collimator carrier 11 is rotated to bring the shielding rods 15 into register with the radioactive sources 2, the rays can be blocked. The shielding rod is made of heavy metal material, for example, tungsten steel or diluted uranium, to enhance its shielding effect.

In the radiating unit of this embodiment, the source carrier 4 is mounted in a shielding case 3. The collimators of different aperture diameter and the shielding rod 15 corresponding to a certain radioactive source 2 are arranged in the manner shown in Figure 1. In the middle of the collimator carrier 11 is a mandrel 10, one end of which is connected with the collimator carrier driving unit 6 via a gear 7 and the other end is fixed on the collimator carrier 11. Source carrier 4 and collimator carrier 11 are connected by means of a high precision bearing 13 so that the collimator carrier driving unit 6 can be precisely positioned so as to guarantee that the selected set of collimators 1 on collimator carrier 11 will accurately come into register with the radioactive sources 2. In the present embodiment, on the source carrier 4 is mounted a sleeve 5 which can be rotated relative to the shielding case 3 and the mandrel 10. One end of the sleeve 5 is secured on the source carrier 4 and the other end is connected to the source carrier driving unit 8 via a gear 9. The source carrier 4 and the shielding case 3 are connected by means of a high precision bearing 12. The source carrier 4 is normally rotated at a low speed state to perform the function of rotatory focusing. The source carrier 4 and the collimator carrier 11 can be made to move relative to one another for replacing the collimators and for acting as a shield during non-therapy hours. During the process of therapy, the source carrier 4 and the collimator carrier 11 are relatively stationary. To guarantee the precision of focusing, the mandrel 10 and the sleeve 5 are each provided with a rotation encoder to measure and determine the relative positions of the source carrier 4 and the collimator carrier 11. An AC servo motor, a harmonic reducer, and an encoder are provided for use with the collimator carrier driving unit 6 and the source carrier driving unit 8. The rotations of the collimator carrier and the source carrier are precisely controlled by means of a double servo control system to effect the real time tracking so that the required focusing at the focus 14 by beams incident from different rotational directions can be achieved.

The gear 7 and gear 9 of the present invention could be replaced by other similar driving members.

## Claims

1. A process for converting the beam diameter of radioactive rays, in which a set of radioactive sources (2) is mounted in the source carrier (4) in such a manner that they are radially in alignment with a common focus (14) on the central axis of the source carrier, and several sets of collimators (1) of different aperture diameter are disposed for alignment with the radioactive sources (2), characterized in that said sets of collimators (1) of different aperture diameter are distributed over a collimator carrier (11) symmetrically about a central axis, the rule of distribution of the sets of the collimators (1) over the collimator carrier (11) being the same as that of the radioactive sources (2); in that the collimator carrier (11) is in an inner cavity of the source carrier (4) and is able to rotate relative to the source carrier (4) about the central axis of the source carrier (4), in order to reposition the collimators; in that when a set of collimators (1) of a certain aperture diameter is being selected, the collimator carrier (11) is made to rotate so that said set of the collimators comes into register with the radioactive sources (2); and in that when a set of collimators of another aperture diameter is selected, the collimator carrier is rotated again until another set of selected collimators comes into register with the radioactive sources (2).

2. The process according to claim 1 characterized in that the centre line of the radioactive source (2) on the source carrier (4) and the centre lines of the corresponding collimators (1) of different aperture diameter are located along a common theoretical conical surface and arranged at a given angle.

3. The process according to claim 1 or 2, characterized in that on said collimator carrier (11) is also mounted a set of shielding rods (15) the rule of distribution of which is the same as that of the radioactive sources (2), said shielding rods being made of heavy metal; and in that the collimator carrier is rotated to bring the shielding rods (15) in register with the radioactive sources (2) during non-therapy hours.

4. The process according to claim 3, characterized in that said source carrier (4) and collimator carrier (11) are of hemi-spherical shape.

5. A medical radioactive source radiating unit for carrying out the beam diameter converting process as claimed in claim 1, comprising a source carrier (4); a shielding case (3); a collimator carrier (11); collimator carrier driving member (7); a collimator carrier driving unit (6); and a set of radioactive sources (2) in the source carrier (4) in radial alignment with a common focus (14) on the central axis of the source carrier; characterized in that the collimator carrier (11) is in the inner cavity of the source carrier (4); and in that on the collimator carrier (11) is a plurality of sets of collimators (1) of different aperture diameter distributed in correspondence with the distribution of radioactive sources (2) on the source carrier (4); and in that a mandrel (10) is mounted in the middle of the collimator carrier (11) and has one end connected with the collimator carrier driving unit (6) via the driving member (7) and the other end fixed to the collimator carrier (11).

6. The radiating unit according to claim 5, characterized in that said collimator carrier (11) is connected with the source carrier (4) by a bearing (13).

7. The radiating unit according to claim 5 or 6, characterized in that said source carrier (4) is connected with said shielding case (3) by a bearing (12); in that a sleeve (5) is mounted on said source carrier (4) and can be rotated relative to said shielding case (3) and said mandrel (10), one end of said sleeve being secured to said source carrier (4) and the other end connected to a source carrier driving unit (8) via a gear (9).

8. The radiating unit according to any one of claims 5 to 7, characterized in that said collimator carrier driving member (7) is a gear.
